Europäisches Patentamt

European Patent Office

Office européen des brevets

(19)

(11) **EP 1 600 149 A1**

(12) **DEMANDE DE BREVET EUROPEEN**

(43) Date de publication:
**30.11.2005 Bulletin 2005/48**

(51) Int Cl.⁷: **A61K 7/13**

(21) Numéro de dépôt: **05291149.2**

(22) Date de dépôt: **27.05.2005**

(84) Etats contractants désignés:
**AT BE BG CH CY CZ DE DK EE ES FI FR GB GR
HU IE IS IT LI LT LU MC NL PL PT RO SE SI SK TR**
Etats d'extension désignés:
**AL BA HR LV MK YU**

(30) Priorité: **28.05.2004 FR 0405834**

(71) Demandeur: **L'OREAL
75008 Paris (FR)**

(72) Inventeurs:
• **Brun, Gaelle
75015 Paris (FR)**
• **Vic, Gabin
60280 Venette (FR)**

(74) Mandataire: **Fevrier, Murielle Françoise E.
L'OREAL - D.I.P.I.
25-29 Quai Aulagnier
92600 Asnières (FR)**

(54) **Composition pour la coloration des fibres kératiniques comprenant un pigment et des polymères aptes à réagir l'un avec l'autre pour former des liaisons covalentes**

(57) La présente invention a pour objet une composition pour la coloration des fibres kératiniques comprenant au moins un polymère PA et au moins un polymère PB, les polymères PA et PB étant aptes à réagir l'un avec l'autre pour former des liaisons covalentes, et au moins un pigment, les procédés de coloration des fibres kératiniques mettant en oeuvre ce pigment et ces polymères, ainsi que l'utilisation de ce pigment et de ces polymères pour la coloration des fibres kératiniques.

La présente invention permet l'obtention d'une coloration visible sur support foncé sans qu'il soit nécessaire d'éclaircir ou de décolorer les fibres kératiniques et résistant bien aux diverses agressions que peuvent subir les cheveux, en particulier aux shampooings et aux frottements.

EP 1 600 149 A1

**Description**

La présente invention a pour objet une composition pour la coloration des fibres kératiniques, et en particulier des fibres kératiniques humaines telles que les cheveux, comprenant au moins un polymère PA et au moins un polymère PB, les polymères PA et PB étant aptes à réagir l'un avec l'autre pour former des liaisons covalentes, et au moins un pigment.

Dans le domaine de la teinture des fibres kératiniques, il existe deux modes de coloration qui présentent chacun leurs avantages et leurs inconvénients.

La *coloration directe* ou *coloration semi-permanente* consiste à teindre les fibres kératiniques avec des compositions tinctoriales contenant des colorants directs. Ces colorants sont des molécules colorées et colorantes ayant une affinité pour les fibres kératiniques. Ils sont appliqués sur les fibres kératiniques pendant un temps nécessaire à l'obtention de la coloration désirée, puis rincés.

Les colorants classiques qui sont utilisés sont en particulier des colorants du type nitré benzénique, anthraquinonique, nitropyridinique, azoïque, xanthénique, acridinique azinique, triarylméthane ou des colorants naturels.

Il a par ailleurs déjà été proposé l'utilisation de pigments, comme dans la demande de brevet FR 2 741 530, qui préconise l'utilisation pour la coloration des fibres kératiniques d'une composition comprenant au moins une dispersion de particules de polymère filmogène comportant au moins une fonction acide et au moins un pigment dispersé dans la phase continue de ladite dispersion.

Les colorations obtenues par ce mode de coloration présentent l'inconvénient d'avoir une faible résistance aux shampooings.

La *coloration d'oxydation* ou *coloration permanente* consiste à teindre les fibres kératiniques avec des compositions tinctoriales contenant des précurseurs de colorant d'oxydation, appelés généralement bases d'oxydation, tels que des ortho- ou para-phénylènediamines, des ortho- ou para-aminophénols et des composés hétérocycliques tels que des dérivés de diaminopyrazole. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les métadiphénols et certains composés hétérocycliques tels que des composés indoliques.

Les nuances obtenues avec la coloration d'oxydation présentent en général une assez bonne ténacité aux shampooings.

La difficulté avec ces deux modes de coloration, la *coloration directe* ou *coloration semi-permanente* d'une part, et la *coloration d'oxydation* ou *coloration permanente* d'autre part, est que si l'on souhaite colorer un support foncé, il est nécessaire de décolorer ou d'éclaircir le support afin que la couleur apportée soit visible.

La décoloration ou l'éclaircissement des fibres kératiniques peut être réalisé à l'aide d'un agent oxydant. Parmi les agents oxydants classiquement utilisés, on peut citer le peroxyde d'hydrogène ou des composés susceptibles de produire du peroxyde d'hydrogène par hydrolyse, comme par exemple le peroxyde d'urée ou les persels tels que les perborates, les persulfates, les percarbonates.

L'utilisation d'agents oxydants, que ce soit pour éclaircir et / ou pour oxyder les colorants d'oxydation, présente l'inconvénient d'entraîner une dégradation non négligeable des fibres kératiniques et d'altérer leurs propriétés cosmétiques. Les cheveux ont tendance à devenir rêches, plus difficilement démêlables et plus fragiles.

Le but de la présente invention est de fournir de nouvelles compositions pour la coloration des fibres kératiniques, et en particulier des fibres kératiniques humaines telles que les cheveux, qui permettent d'obtenir des colorations qui sont visibles sur support foncé sans qu'il soit nécessaire d'éclaircir ou de décolorer les fibres et qui présentent une bonne résistance aux shampooings.

Ce but est atteint avec la présente invention qui a pour objet une composition pour la coloration des fibres kératiniques comprenant, dans un milieu cosmétiquement acceptable, au moins un polymère PA portant au moins une fonction chimique A et au moins un polymère PB portant au moins une fonction chimique B, lesdites fonctions chimiques A et B étant aptes à former ensemble des liaisons covalentes, et au moins un pigment.

La composition conforme à la présente invention conduit à une coloration visible sur support foncé sans qu'il soit nécessaire d'éclaircir ou de décolorer les fibres kératiniques, et par conséquent sans dégradation physique des fibres kératiniques. Cette coloration est de plus résistante aux diverses agressions que peuvent subir les cheveux telles que les shampooings, les frottements, la lumière, les intempéries, la sueur et les déformations permanentes. Ces propriétés sont particulièrement remarquables en ce qui concerne la résistance de la coloration vis à vis des shampooings et des frottements. La coloration est obtenue dans des nuances variées, elle est chromatique, puissante, esthétique et peu sélective.

La présente invention a aussi pour objet un procédé de coloration des fibres kératiniques mettant en oeuvre au moins un polymère PA portant au moins une fonction chimique A et au moins un polymère PB portant au moins une fonction chimique B, lesdites fonctions A et B étant aptes à réagir l'une avec l'autre pour former des liaisons covalentes, et au moins un pigment.

Un autre objet de la présente invention est l'utilisation pour la coloration des fibres kératiniques d'au moins un polymère PA portant au moins une fonction chimique A et au moins un polymère PB portant au moins une fonction chimique B, lesdites fonctions A et B étant aptes à réagir l'une avec l'autre pour former des liaisons covalentes, et d'au moins un pigment.

Conformément à l'invention, pour déterminer si les fonctions chimiques A et B des polymères PA et PB réagissent l'une avec l'autre pour former des liaisons covalentes, par exemple par réaction de substitution, réaction d'addition sur des doubles ou triples liaisons carbone-carbone ou carbone-hétéroatome, réaction d'ouverture de cycles, on met en oeuvre le test décrit ci après, les opérations (1) à (4) étant conduites à la température ambiante :

(1) On applique 0,25 gramme d'une solution du polymère PA dans un solvant choisi parmi l'eau, l'éthanol, les esters, les cétones et de préférence l'eau, avec une teneur en poids du polymère par rapport au poids total de la solution comprise entre 10 et 50 %, sur une lame de verre ;

(2) On laisse le solvant du polymère PA s'évaporer, jusqu'à l'obtention d'un dépôt sec ;

(3) On applique sur la lame déjà recouverte par le polymère PA 0,25 gramme d'une solution du polymère PB dans un solvant choisi parmi l'eau, l'éthanol, les esters, les cétones et de préférence l'eau, avec une teneur en poids du polymère PB par rapport au poids total de la solution comprise entre 10 et 50 % ;

(4) On laisse le solvant du polymère PB s'évaporer jusqu'à l'obtention d'un dépôt sec ;

(5) On place la lame de verre recouverte par les polymères PA et PB dans une enceinte à une température de 130 °C, et pendant une durée de 120 minutes ;

(6) Dans le cas où les solvants utilisés dans les points (1) et (3) sont différents, on répète une seconde fois les points (1) à (5);

(7) Le solide obtenu est entièrement immergé dans 10 grammes du solvant utilisé au point (1) ;

(8) Dans le cas où les solvants utilisés dans les points (1) et (3) sont différents, on immerge le solide obtenu à partir du point (6) dans 10 grammes du solvant utilisé au point (3) ;

(9) Les polymères PA et PB réagissent l'un avec l'autre pour former des liaisons covalentes si au moins 50 % en poids du solide ne se dissout pas au bout de 3 jours, à température ambiante et sans agitation, dans les solvants des points (7) et (8).

Dans les étapes (1) et (3), lorsque les solutions sont aqueuses, elles sont, de préférence, ajustées au pH d'utilisation des compositions de l'invention.

On peut aussi vérifier la formation de liaisons covalentes entre les polymères PA et PB à l'aide de techniques de caractérisation classiques, telles que la spectroscopie Infra-Rouge ou la spectroscopie de photoélectrons X (XPS).

Dans le cadre de l'invention, les fonctions chimiques A peuvent réagir avec les fonctions chimiques B, soit spontanément, soit par une activation par la température, le pH, un co-réactif ou un catalyseur chimique ou biochimique, comme une enzyme.

Selon un mode de réalisation particulier de l'invention, les fonctions chimiques A sont choisies parmi les fonctions suivantes :

- époxyde,
- aziridine,
- vinyle et vinyle activée, en particulier, acrylonitrile, esters acrylique et métacrylique, acide et esters crotonique, acide et esters cinnamique, styrène et dérivés, butadiène, éthers de vinyle, vinylcétone, esters maléiques, vinyl sulfones, maléimides,
- anhydride, chlorure d'acide et esters d'acide carboxylique,
- aldéhydes,
- acétals, hémi-acétals,
- aminals, hémi aminals,
- cétones, alpha-hydroxycétones, alpha-halocétones,
- lactones, thiolactones,
- isocyanate,
- thiocyanate,
- imines,
- imides, en particulier, succinimide, glutimide,
- N-hydroxysuccinimide esters,
- imidates,

- thiosulfate,
- oxazine et oxazoline,
- oxazinium et oxazolinium,
- halogénures d'alkyle en $C_1$ à $C_{30}$ ou d'aryle ou aralkyle en $C_6$ à $C_{30}$ de formule RX avec X = I, Br, Cl,
- halogénure de cycle insaturé, carboné ou hétérocycle, notamment les chlorotriazine, chloropyrimidine, chloroquiinoxaline, chlorobenzotriazole,
- halogénure de sulfonyle de formule $RSO_2Cl$ ou F, R étant un alkyle en $C_1$ à $C_{30}$.

De préférence, les fonctions chimiques A sont choisies parmi les fonctions chimiques A sont choisies parmi les fonctions anhydride, époxyde, chlorotriazine, aldéhyde, thiosulfate.

Selon un mode de réalisation particulier de l'invention, les fonctions chimiques B sont choisies parmi les fonctions hydroxy, amines primaires et secondaires, les thiols, les acides carboxyliques.

Les polymères selon l'invention peuvent contenir des fonctions chimiques autres que les fonctions A et B.

Par polymère, on entend un composé comportant au moins 2 motifs de répétition enchaînés par des liaisons covalentes.

Les polymères PA et PB peuvent être d'origine naturelle, modifiés chimiquement ou non, comme par exemple les polysaccharides tels que la cellulose, le dextrane, le chitosane, le guar et leurs dérivés hydroxyalkylés, carboxyméthylés, aminés ou thiolés, ou leurs dérivés à fonction aldéhyde ou époxy.

Ils peuvent être des polymères synthétiques tels que les polyacrylates, les polyméthacrylates, les polyvinyles, les polyesters, les polyéthers, les polyamides, les polyuréthanes, les polydiméthylsiloxanes, les polypeptides. Ces polymères peuvent être synthétisés selon des méthodes connues dans la littérature.

Ces polymères peuvent se présenter sous tout type de topologie : chaîne linéaire, ramifiée, en étoile ou hyperbranchée, tels que les dendrimères, chaînes séquencées, statistiques ou alternées.

Les fonctions chimiques A et B peuvent être présentes sur la chaîne polymère, en bout de chaîne, greffées le long de la chaîne principale ou des chaînes secondaires, sur les branches des polymères en étoile ou hyperbranchés.

Dans un mode de réalisation particulier de l'invention, le polymère PA possède au moins deux fonctions chimiques A identiques et le polymère PB possède au moins deux fonctions chimiques B identiques, afin de se lier avec au moins deux autres polymères.

De préférence, le ou les polymères PA sont choisis parmi les polymères suivants :

- les polymères contenant le motif anhydride, notamment maléique,
- les polymères contenant le groupe époxyde et / ou aldéhyde,
- les polysaccharides naturels ou modifiés présentant des fonctions aldéhydes,
- les polysaccharides naturels ou modifiés présentant des fonctions époxy,
- les polymères naturels ou synthétiques à fonctions acide carboxylique.

Le ou les polymères PB sont de préférence choisis parmi les polymères synthétiques ou naturels à fonctions OH ou $NH_2$ ou SH ou COOH.

A titre d'exemples de polymères synthétiques ou naturels à fonctions OH ou $NH_2$ ou SH ou COOH, on peut citer :

- les dendrimères à terminaisons OH, ou $NH_2$ ou SH ou COOH de une ou plusieurs générations,
- les polymères synthétiques à fonction hydroxyle, tes que les alcools polyvinyliques,
- les polyéthylèneimines,
- les polyéthylèneimine thiols, obtenus par réaction de polyéthylèneimines sur la ?-butyrolactone, tels que ceux décrits dans le brevet L'OREAL FR 2 772 770,
- les polyacides aminés présentant des groupes OH, ou $NH_2$ ou SH ou COOH libres, par exemple la polylysine,
- les polysaccharides naturels ou modifiés présentant des fonctions OH, ou $NH_2$ ou SH ou COOH, comme par exemple le chitosane et ses dérivés, tels que les carboxyméthyl-chitosanes ou les amino-dextranes.

Selon la présente invention, les associations de polymères PA et PB suivantes sont préférées :

- un polymère PB choisi parmi les dendrimères à terminaisons OH, ou $NH_2$ ou SH ou COOH de une ou plusieurs générations en association avec un polymère PA choisi parmi les polymères contenant le motif anhydride, notamment maléique, ou les polymères contenant le groupe époxyde et / ou aldéhyde,
- un polymère PB choisi parmi les polymères synthétiques à fonction hydroxyle, tels les alcools polyvinyliques, en association avec un polymère PA choisi parmi les polymères contenant le motif anhydride, notamment maléique ou les polymères contenant le groupe époxyde,

- un polymère PB choisi parmi les polyéthylèneimines en association avec un polymère PA choisi parmi les polymères contenant le motif anhydride, notamment maléique ou les polymères contenant le groupe époxyde et / ou aldéhyde,
- un polymère PB choisi parmi les polyéthylèneimine thiols, obtenus par réaction de polyéthylèneimines sur la γ-butyrolactone, tels que ceux décrits dans le brevet L'OREAL FR 2 772 770, en association avec un polymère PA choisi parmi les polymères contenant le motif anhydride, notamment maléique ou les polymères contenant le groupe époxyde et / ou aldéhyde,
- un polymère PB choisi parmi les polyacides aminés présentant des groupes OH, ou $NH_2$ ou SH ou COOH libres, par exemple la polylysine en association avec un polymère PA choisi parmi les polymères contenant le motif anhydride, notamment maléique ou les polymères contenant le groupe époxyde et / ou aldéhyde,
- un polymère PB choisi parmi les polysaccharides naturels ou modifiés présentant des fonctions OH, ou $NH_2$ ou SH ou COOH en association avec un polymère PA choisi parmi les polymères contenant le motif anhydride, notamment maléique ou les polymères contenant le groupe époxyde et / ou aldéhyde,
- un polymère PA choisi parmi les polysaccharides naturels ou modifiés présentant des fonctions aldéhydes en association avec un polymère PB choisi parmi les polymères synthétiques ou naturels à fonctions OH ou $NH_2$ ou SH ou COOH comme par exemple la polyéthylèneimine, la polylysine, le chitosane et ses dérivés, tels que les carboxyméthyl-chitosanes ou les amino-dextranes,
- un polymère PA choisi parmi les polysaccharides naturels ou modifiés présentant des fonctions époxy en association avec un polymère PB choisi parmi les polymères synthétiques ou naturels à fonctions OH ou $NH_2$ ou SH ou COOH comme par exemple la polyéthylèneimine, la polylysine, le chitosane et ses dérivés, tels que les carboxyméthyl-chitosanes ou les amino-dextranes,
- un polymère PA choisi parmi les polymères naturels ou synthétiques à fonctions acide carboxylique en association avec un polymère PB choisi parmi les polymères synthétiques ou naturels à fonctions OH, $NH_2$ ou SH, en présence d'un carbodiimide, ou d'un acide, d'une base ou d'une enzyme, comme les estérase, lipase, protéase.

Par exemple, et sans limitation, on peut citer, à titre de polymères PA, les polymères énumérés ci-après :

- Copolymère méthyl-vinyl éther / anhydride maléique, en particulier commercialisé par exemple par ISP sous le nom de Gantrez,
- PolyMethacrylate de glycidyle, en particulier commercialisé par Polysciences,
- Polydimethylsiloxane de glycidyle, en particulier commercialisé par Shinetsu (référence X-2Z-173 FX ou DX),
- Polyamidoamine époxy, par exemple commercialisé par Hercules sous le nom de Delsette 101, le Kymène 450 de chez Hercules,
- Epoxy-dextrane,
- Polysaccharides polyaldéhydes obtenus par oxydation de polysaccharides par NaIO4 (méthodes connues, Bioconjugate Techniques ; Hermanson GT, Academic Press, 1996),

et, à titre de polymères PB, les polymères énumérés ci-après :

- Dendrimère PAMAM, en particulier commercialisé par Dendritech, DSM, Sigma-Aldrich (STARBURST, PAMAM DENDRIMER, G(2, O) de chez DENDRITECH),
- Dendrimère à fonctions hydroxy, en particulier commercialisé par Perstorp, DSM, (exemple : HBP TMP core 2 Generation PERSTORP),
- PEI (polyéthylène-imine), en particulier commercialisé par BASF, sous le nom de Lupasol,
- PEI-Thiol,
- Polylysine, en particulier commercialisé par Chisso,
- HP cellulose, tel que KLUCELEF de chez AQUALON,
- Amino-dextrane, par exemple commercialisé par Carbomer,
- Amino-cellulose, par exemple ceux décrits dans WO 01/25283 de BASF,
- PVA (polyvinylacétal), par exemple AIRVOL 540 de chez AIRPRODUCTS CHEMICAL,
- Amino PVA, par exemple commercialisé par Carbomer,
- Chitosane,
- CM ou HP Dextrane, par exemple commercialisé par Fluka,
- CM ou HP Chitosane, par exemple commercialisé par Fluka.

Toutes les associations d'au moins un polymère PA à fonctions chimiques A et d'au moins un polymère PB à fonctions chimiques B sont citées à titre d'exemples, sans limitation.
Par pigment, on entend toute entité organique et / ou minérale dont la solubilité dans l'eau est inférieure à 0,01 % à 20 °C, présentant une absorption entre 350 et 700 nm, de préférence une absorption avec un maximum.

Les pigments conformes à la présente invention sont choisis parmi tous les pigments organiques et / ou minéraux, notamment ceux qui sont décrits dans l'encyclopédie de technologie chimique de Kirk-Othmer et dans l'encyclopédie de chimie industrielle de Ullmann.

Les pigments conformes à l'invention peuvent se présenter sous forme de poudre ou de pâte pigmentaire. Ils peuvent être enrobés ou non enrobés.

Les pigments conformes à l'invention peuvent par exemple être choisis parmi les pigments blancs ou colorés, les laques, les pigments à effets spéciaux tels que les nacres ou les paillettes, et leurs mélanges.

A titre d'exemples de pigments minéraux blancs ou colorés, on peut citer le dioxyde de titane, traité ou non traité en surface, les oxydes de zirconium ou de cérium, les oxydes de fer ou de chrome, le violet de manganèse, le bleu outremer, l'hydrate de chrome et le bleu ferrique.

A titres d'exemples de pigments organiques blancs ou colorés, on peut citer les composés nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.

En particulier, les pigments organiques blancs ou colorés peuvent être choisis parmi le carmin, le noir de carbone, le noir d'aniline, le jaune azo, la quinacridone, le bleu de phtalocyanine, le rouge sorgho, les pigments bleus codifiés dans le Color Index sous les références CI 42090, 69800, 69825, 73000, 74100, 74160, les pigments jaunes codifiés dans le Color Index sous les références CI 11680, 11710, 15985, 19140, 20040, 21100, 21108, 47000, 47005, les pigments verts codifiés dans le Color Index sous les références CI 61565, 61570, 74260, les pigments oranges codifiés dans le Color Index sous les réfénces CI 11725, 15510, 45370, 71105, les pigments rouges codifiés dans le Color Index sous les références CI 12085, 12120, 12370, 12420, 12490, 14700, 15525, 15580, 15620, 15630, 15800, 15850, 15865, 15880, 17200, 26100, 45380, 45410, 58000, 73360, 73915, 75470, les pigments obtenus par polymérisation oxydante de dérivés indoliques, phénoliques tels qu'ils sont décrits dans le brevet FR 2 679 771.

On peut utiliser des pâtes pigmentaires de pigment organique telles que les produits vendus par la société HOECHST sous le nom :

- JAUNE COSMENYL IOG : Pigment YELLOW 3 (CI 11710) ;
- JAUNE COSMENYL G : Pigment YELLOW 1 (CI 11680) ;
- ORANGE COSMENYL GR : Pigment ORANGE 43 (CI 71105) ;
- ROUGE COSMENYL R : Pigment RED 4 (CI 12085) ;
- CARMIN COSMENYL FB : Pigment RED 5 (CI 12490) ;
- VIOLET COSMENYL RL : Pigment VIOLET 23 (CI 51319) ;
- BLEU COSMENYL A2R : Pigment BLUE 15.1 (CI 74160) ;
- VERT COSMENYL GG : Pigment GREEN 7 (CI 74260) ;
- NOIR COSMENYL R : Pigment BLACK 7 (CI 77266).

Les pigments conformes à l'invention peuvent aussi être sous forme de pigments composites tels qu'ils sont décrits dans le brevet EP 1 184 426. Ces pigments composites peuvent être composés notamment de particules comportant :

- un noyau inorganique,
- au moins un liant assurant la fixation des pigments organiques sur le noyau, et
- au moins un pigment organique recouvrant au moins partiellement le noyau.

Par laque, on entend les colorants adsorbés sur des particules insolubles, l'ensemble ainsi obtenu restant insoluble lors de l'utilisation. Les substrats inorganiques sur lesquels sont adsorbés les colorants sont par exemple l'alumine, la silice, le borosilicate de calcium et de sodium ou le borosilicate de calcium et d'aluminium, et l'aluminium. Parmi les colorants organiques, on peut citer le carmin de cochenille.

A titre d'exemples de laques, on peut citer les produits connus sous les dénominations suivantes : D & C Red 21 (CI 45 380), D & C Orange 5 (CI 45 370), D & C Red 27 (CI 45 410), D & C Orange 10 (CI 45 425), D & C Red 3 (CI 45 430), D & C Red 7 (CI 15 850:1), D & C Red 4 (CI 15 510), D & C Red 33 (CI 17 200), D & C Yellow 5 (CI 19 140), D & C Yellow 6 (CI 15 985), D & C Green (CI 61 570), D & C Yellow 1 O (CI 77 002), D & C Green 3 (CI 42 053), D & C Blue 1 (CI 42 090).

Par pigments à effets spéciaux, on entend les pigments qui créent d'une manière générale une apparence colorée (caractérisée par une certaine nuance, une certaine vivacité et une certaine clarté) non uniforme et changeante en fonction des conditions d'observation (lumière, température, angles d'observation...). Ils s'opposent par-là même aux pigments blancs ou colorés qui procurent une teinte uniforme opaque, semi-transparente ou transparente classique.

A titre d'exemples de pigments à effets spéciaux, on peut citer les pigments nacrés blancs tels que le mica recouvert de titane, ou d'oxychlorure de bismuth, les pigments nacrés colorés tels que le mica recouvert de titane et d'oxydes de fer, le mica recouvert de titane et notamment de bleu ferrique ou d'oxyde de chrome, le mica recouvert de titane et d'un pigment organique tel que défini précédemment ainsi que les pigments nacrés à base d'oxychlorure de bismuth.

On peut également citer les pigments à effet interférentiel non fixés sur un substrat comme les cristaux liquides (Helicones HC de Wacker), les paillettes holographiques interférentielles (Geometric Pigments ou Spectra f/x de Spectratek). Les pigments à effets spéciaux comprennent aussi les pigments fluorescents, que ce soit les substances fluorescentes à la lumière du jour ou qui produisent une fluorescence ultraviolette, les pigments phosphorescents, les pigments photochromiques, les pigments thermochromiques et les quantum dots, commercialisés par exemple par la société Quantum Dots Corporation.

Les quantum dots sont des nanoparticules semi conductrices luminescentes capables d'émettre, sous excitation lumineuse, un rayonnement présentant une longueur d'onde comprise entre 400 nm et 700 nm. Ces nanoparticules sont connues de la littérature. En particulier, elles peuvent être fabriqués selon les procédés décrits par exemple dans le US 6 225 198 ou US 5 990 479, dans les publications qui y sont citées, ainsi que dans les publications suivantes : Dabboussi B.O. et al "(CdSe)ZnS core-shell quantum dots : synthesis and characterisation of a size series of highly luminescent nanocristallites" *Journal of phisical chemistry B, vol 101, 1997, pp 9463-9475.* et Peng, Xiaogang et al, "Epitaxial Growth of highly Luminescent CdSe/CdS core/shell nanocrystals with photostability and electronic accessibility" *Journal of the American Chemical Society, vol 119, N° 30, pp 7019-7029.*

Les pigments conformes à l'invention sont de préférence des pigments colorés.

La variété des pigments mis en jeu permet d'obtenir une riche palette de couleurs, ainsi que des effets optiques particuliers tels que des effets métalliques, interférentiels.

La taille d'un pigment autre que les nacres en solution est généralement comprise entre 10 nm et 10 μm, de préférence entre 50 nm et 5 μm, et encore plus préférentiellement entre 100 nm et 3 μm. La taille d'une nacre en solution est généralement comprise entre 1 et 200 μm, de préférence entre 1 et 80 μm, et encore plus préférentiellement entre 1 et 50 μm.

Selon un mode de réalisation particulier de l'invention, le ou les pigments sont en dispersion dans la composition conforme à l'invention.

Le ou les polymères PA et PB sont chacun généralement présents dans la composition conforme à l'invention à des concentrations comprises entre 0,05 et 50 % en poids, de préférence entre 0,1 et 20 % en poids, et encore plus préférentiellement entre 0,25 et 10 % en poids par rapport au poids total de la composition.

Le ou les pigments sont chacun généralement présents dans la composition conforme à l'invention à des concentrations comprises entre 0,05 et 80 % en poids, de préférence entre 0,1 et 60 % en poids, et encore plus préférentiellement entre 0,25 et 40 % en poids par rapport au poids total de la composition.

La composition selon la présente invention peut de plus comprendre au moins un agent activateur.

De préférence, le ou les agents activateurs sont choisis parmi les modificateurs de pH, les co-réactifs et les catalyseurs.

Avantageusement, on choisit :

- comme modificateur de pH, des acides ou des bases, de nature minérale ou organique ;
- comme co-réactif, du carbodiimide, des oxydants ou des réducteurs ;
- comme catalyseur, des enzymes telles que la transglutaminase.

Selon un mode de réalisation particulier de l'invention, le ou les agents activateurs sont en dispersion dans la composition conforme à l'invention.

Le ou les agents activateurs sont chacun généralement présents à des concentrations comprises entre 0,05 et 30 % en poids, de préférence entre 0,1 et 20 % en poids, et encore plus préférentiellement entre 0,25 et 10 % en poids par rapport au poids total de la composition.

La composition conforme à l'invention peut de plus comprendre des charges.

Par charges, il faut comprendre des particules incolores ou blanches, minérales ou de synthèse, lamellaires ou non lamellaires. Les charges peuvent être présentes à raison de 0 à 80 % en poids, par rapport au poids total de la composition, de préférence de 0,01 à 60 % en poids, et mieux de 0,02 % à 40 % en poids. On peut notamment citer le talc, le stéarate de zinc, le kaolin, les poudres de polyamide (Nylon®) (Orgasol de chez Atochem), les poudres de polyéthylène, les poudres de polymères de tétrafluoroéthylène (TéflonB), l'amidon, le nitrure de bore, des microsphères polymériques telles que celles de chlorure de polyvinylidène / acrylonitrile comme l'Expancel (Nobel Industrie), de copolymères d'acide acrylique (PolytrapB de la société Dow Corning) et les microbilles de résine de silicone (TospearlsO de Toshiba, par exemple), les organopolysiloxanes élastomères.

La composition selon l'invention contient avantageusement, en outre, des additifs cosmétiques conventionnels choisis parmi les polymères fixants, les épaississants, les tensioactifs anioniques, non ioniques, cationiques ou amphotères, les parfums, les conservateurs, les filtres solaires, les protéines, les vitamines, les provitamines, les polymères non fixants anioniques, non ioniques, cationiques ou amphotères, les huiles minérales, végétales ou synthétiques, les céramides, les pseudocéramides, les silicones volatiles ou non, linéaires ou cycliques, modifiées ou non, les agents régulateurs de pH, les oxydants, les réducteurs, les inhibiteurs, les catalyseurs, les colorants d'oxydation tels que les bases d'oxydation et les coupleurs, les colorants directs autres que les pigments et tout autre additif classiquement utilisé dans les compositions cosmétiques.

Le procédé selon la présente invention consiste à appliquer sur les fibres kératiniques :

- au moins un polymère PA et au moins un polymère PB tels que définis précédemment ;
- et au moins un pigment tel que défini précédemment.

Le ou les polymères PA, le ou les polymères PB et le ou les pigments peuvent être appliqués sur les fibres kératiniques à partir de plusieurs compositions contenant le ou les polymères PA, le ou les polymères PB et le ou les pigments, seuls ou en mélange, ou à partir d'une seule composition contenant le ou les polymères PA, le ou les polymères PB et le ou les pigments.

Selon un mode de réalisation particulier de l'invention, on applique sur les fibres kératiniques une composition (A) comprenant, dans un milieu cosmétiquement acceptable, le ou les polymères PA et le ou les polymères PB, ainsi que le ou les pigments.

Selon un autre mode de réalisation particulier de l'invention, on applique sur les fibres kératiniques une composition (B) comprenant, dans un milieu cosmétiquement acceptable, le ou les pigments, et une composition (C) comprenant, dans un milieu cosmétiquement acceptable, le ou les polymères PA et le ou les polymères PB, l'ordre d'application des compositions (B) et (C) étant indifférent.

Selon un autre mode de réalisation particulier de l'invention, on applique sur les fibres kératiniques une composition (B) comprenant, dans un milieu cosmétiquement acceptable, le ou les pigments, une composition (D) comprenant, dans un milieu cosmétiquement acceptable, le ou les polymères PA et une composition (E) comprenant, dans un milieu cosmétiquement acceptable, le ou les polymères PB, l'ordre d'application des compositions (B), (D) et (E) étant indifférent.

Selon un autre mode de réalisation particulier de l'invention, on applique sur les fibres kératiniques une composition (F) comprenant, dans un milieu cosmétiquement acceptable, le ou les polymères PA et le ou les pigments et une composition (E) comprenant, dans un milieu cosmétiquement acceptable, le ou les polymères PB, l'ordre d'application des compositions (F) et (E) étant indifférent.

Selon un autre mode de réalisation particulier de l'invention, on applique sur les fibres kératiniques une composition (D) comprenant, dans un milieu cosmétiquement acceptable, le ou les polymères PA et une composition (G) comprenant, dans un milieu cosmétiquement acceptable, le ou les polymères PB et le ou les pigments, l'ordre d'application des compositions (D) et (G) étant indifférent.

Selon un mode de réalisation préféré de l'invention, la composition comprenant le ou les pigments est appliquée avant la ou les compositions comprenant le ou les polymères PA et / ou le ou les polymères PB.

Selon un mode de réalisation particulier de l'invention, on augmente la température pour activer la réaction entre le ou les polymères PA et le ou les polymères PB. On peut augmenter la température jusqu'à 180 ou 200 °C.

Selon un autre mode de réalisation particulier de l'invention, on applique sur les fibres kératiniques au moins un agent activateur tel que défini précédemment pour activer la réaction entre le ou les polymères PA et le ou les polymères PB.

Par exemple, le ou les agents activateurs peuvent être présents dans l'une ou l'autre ou dans plusieurs des compositions appliquées sur les fibres kératiniques ou dans une composition supplémentaire, auquel cas l'ordre d'application des différentes compositions sur les fibres kératiniques est indifférent.

Les agents activateurs avantageusement choisis sont ceux qui sont décrits ci-dessus.

Selon un mode de réalisation particulier de l'invention, le ou les pigments et le ou les agents activateurs sont en dispersion dans les différentes compositions mises en oeuvre dans le procédé conforme à l'invention les comprenant.

Les différentes compositions mises en oeuvre dans le procédé conforme à l'invention peuvent être appliquées sur des cheveux secs ou humides.

Ces compositions peuvent contenir en outre divers additifs cosmétiques conventionnels tels que définis précédemment.

Un séchage intermédiaire peut être réalisé entre chaque application.

Dans le cas où la réaction entre les différents polymères a lieu spontanément à température ambiante mais leur mélange en solution diluée est stable, on peut appliquer directement sur les fibres kératiniques une solution contenant les polymères dans un solvant volatile cosmétiquement acceptable et au cours de l'évaporation du solvant, la réaction de réticulation a lieu. Le dépôt de polymères devient insoluble et reste sur les fibres kératiniques.

Si le solvant utilisé n'est pas volatile, les fibres kératiniques peuvent être séchées au casque, au sèche cheveux, au fer à lisser.

Dans le cas où la réaction entre les différents polymères nécessite une activation, on peut appliquer le mélange des polymères sur les fibres kératiniques et par augmentation de la température ou ajout d'un agent modificateur de pH, ou ajout d'un co-réactif ou d'un catalyseur, on provoque la réticulation du dépôt. Cet ajout peut avoir lieu immédiatement après le dépôt des polymères sur les fibres kératiniques, après un préséchage des cheveux.

Dans le cas où les deux polymères sont incompatibles en solution, on dépose d'abord sur les fibres kératiniques un des deux polymères via un milieu cosmétiquement acceptable, puis on dépose le ou les polymères susceptibles de réagir avec le premier, via un milieu cosmétiquement acceptable avec ou sans séchage intermédiaire. La réaction chimique peut alors avoir lieu spontanément au cours du séchage ou être déclenchée par un apport de chaleur, une modification de pH, un ajout de co-réactif ou de catalyseur. L'ordre d'application des polymères peut varier.

Les fibres kératiniques peuvent être séchées au casque, au sèche cheveux, au fer à lisser.

Le dépôt réticulé ainsi formé présente l'avantage d'avoir une faible solubilité attendue. En outre, il possède une bonne affinité pour la surface des fibres kératiniques, ce qui garantit une meilleure rémanence de l'ensemble du dépôt.

Lorsque les polymères sont appliqués séparément, le dépôt en couches obtenu peut aussi être avantageux pour conserver les propriétés cosmétiques ou optiques du polymère qui constitue la partie supérieure du dépôt.

Selon les mêmes procédés, il est possible de réaliser des superpositions multiples de couches de polymères qui réticulent entre eux pour atteindre le type de dépôt souhaité (en termes de nature chimique, résistance mécanique, épaisseur, aspect, toucher...).

Le milieu cosmétiquement acceptable, véhiculant le ou les polymères PA et / ou le ou les polymères PB, est choisi de telle sorte que les polymères PA et PB soient aptes à réagir l'un avec l'autre pour former des liaisons covalentes après l'application de la composition cosmétique sur les cheveux.

Dans le cadre de la présente invention, un milieu cosmétiquement acceptable comprend généralement de l'eau et / ou un ou plusieurs solvants cosmétiquement acceptables tels que les alcools, les esters, les cétones ou les silicones volatiles cycliques ou des mélanges eau-solvant(s), ces solvants étant de préférence des alcools en $C_1$-$C_4$.

La présente invention a aussi pour objet un dispositif à plusieurs compartiments contenant au moins deux compositions telles que l'ensemble desdites compositions comprend :

- au moins un polymère PA et au moins un polymère PB tels que définis précédemment ;
- et au moins un pigment tel que défini précédemment.

Selon un mode de réalisation particulier de l'invention, un premier compartiment contient la composition (B) telle que définie précédemment et un deuxième compartiment contient la composition (C) telle que définie précédemment.

Selon un autre mode de réalisation particulier, un premier compartiment contient la composition (B) telle que définie précédemment, un deuxième compartiment contient la composition (D) telle que définie précédemment et un troisième compartiment contient la composition (E) telle que définie précédemment.

Selon un autre mode de réalisation particulier de l'invention, un premier compartiment contient la composition (F) telle que définie précédemment et un deuxième compartiment contient la composition (E) telle que définie précédemment.

Selon un autre mode de réalisation particulier, un premier compartiment contient la composition (D) telle que définie précédemment et un deuxième compartiment contient la composition (G) telle que définie précédemment.

Selon un mode de réalisation particulier de l'invention, l'ensemble desdites compositions comprend de plus au moins un agent activateur tel que défini précédemment.

Par exemple, l'une ou l'autre ou plusieurs des compositions contenues dans le dispositif à plusieurs compartiments peut de plus comprendre au moins un agent activateur.

Le dispositif à plusieurs compartiments peut aussi contenir une composition supplémentaire comprenant, dans un milieu cosmétiquement acceptable, au moins un agent activateur.

Les agents activateurs avantageusement choisis sont ceux qui sont décrits ci-dessus.

La présente invention a également pour objet l'utilisation pour la coloration des fibres kératiniques d'au moins un polymère PA et d'au moins un polymère PB tels que définis précédemment, et d'au moins un pigment tel que défini précédemment.

En particulier, la présente invention a pour objet l'utilisation pour la coloration des fibres kératiniques d'au moins un polymère PA et d'au moins un polymère PB tels que définis précédemment, et d'au moins un pigment tel que défini précédemment, dans laquelle la coloration des fibres kératiniques est résistante aux shampooings.

De préférence, la résistance aux shampooings est telle que le pourcentage de dégradation après 6 shampooings tel que défini ci-après est inférieur à 50 %.

Les exemples non limitatifs suivants permettent d'illustrer l'invention sans en limiter sa frontière.

EXEMPLES

Les exemples suivants ont été réalisés en utilisant le couple de polymères PA, PB Polylysine - Gantrez. Il s'agit dans ce cas de la réticulation entre une fonction amine et une fonction aldéhyde.

Les fibres kératiniques traitées sont des mèches de 1 g de cheveux naturels.

Les différents réactifs utilisés sont les suivants :

- Pigment 1 : Pigment composite de 17 nm avec un coeur de silice et une partie organique constituée de quinacridone (pigment Red 122) préparé par la méthode décrite dans la demande de brevet EP 1 184 426 A2 ;
- Pigment 2: Pigment organique de quinacridone pigment Red 122 (sunfast magenta 122 commercialisé par le groupe Sun) ;
- Pigment 3 : Sel de calcium du rouge lithol B, D&C Red 7 commercialisé par la société Wackherr ;
- Polylysine commercialisée par Chisso ;
- Gantrez S95 commercialisé par ISP ;
- EDC : N-(3-Dimethylaminopropyl)-(N'-ethylcarbodiimide) hydro chloride commercialisé par Fluka ;
- NHS : N-Hydroxysulfosuccinimide sodium Salt commercialisé par Fluka. A partir de ces réactifs, les solutions suivantes ont été réalisées :

- Solution $A_1$ : Solution aqueuse à 10 % en pigment 1.
- Solution $A_2$ : Solution aqueuse à 10 % en pigment 2.
- Solution $A_3$ : Solution aqueuse à 10 % en pigment 3.
- Solution B : Solution aqueuse à 10 % en Polylysine.
- Solution C : Solution aqueuse à 5 % en Gantrez S95.
- Solution $D_1$ : Solution aqueuse à 10 % en pigment 1 et 10 % en Polylysine.
- Solution $D_2$ : Solution aqueuse à 10 % en pigment 2 et 10 % en Polylysine.
- Solution $D_3$ : Solution aqueuse à 10 % en pigment 3 et 10 % en Polylysine.
- Solution $E_1$ : Solution aqueuse à 10 % en pigment 1 et 5 % en Gantrez.
- Solution $E_2$ : Solution aqueuse à 10 % en pigment 2 et 5 % en Gantrez.
- Solution $E_3$ : Solution aqueuse à 10 % en pigment 3 et 5 % en Gantrez.
- Solution F : Solution aqueuse contenant $6.10^{-5}$ mol de EDC et $6.10^{-5}$ mol de NHS.

**Exemple 1 :**

1) 0,5 g de la solution $A_1$ est appliqué sur une mèche de cheveux propres et secs. La mèche est ensuite séchée au casque.
2) 0,5 g de la solution B est ensuite appliqué sur la mèche sèche. Sans séchage intermédiaire 0,5 g de la solution C est appliqué.
3) La mèche est ensuite placée 30 minutes à 140 °C.

Le même mode opératoire est mis en oeuvre pour les solutions $A_2$ et $A_3$.

Avec les solutions $A_1$ et $A_2$, les mèches obtenues sont très colorées en rose. Avec la solution $A_3$, la mèche obtenue est très colorée en rouge.

Les mèches sont ensuite soumises à un test de résistance aux shampooings.

La couleur des cheveux est mesurée à l'aide d'un spectrocolorimètre MINOLTA CM3600d® dans le système CIELab. Dans ce système, L* représente la clarté, a* la teinte et b* la saturation.

La dégradation de la couleur après six shampooings est estimée selon la formule suivante :

$$\%dégradation = 100 * \frac{DE\,6\,Shamp}{DE\,Tém}$$

avec :

$$DE\ tém = \sqrt{(a{*}tém - a{*}\ BN)^2 + (b{*}tém - b{*}\ BN)^2 + (L{*}tém - L{*}\ BN)^2}$$

et

$$DE\ 6Shamp = \sqrt{(a{*}tém - a{*}\ 6Sh)^2 + (b{*}tém - b{*}\ 6Sh)^2 + (L{*}tém - L{*}\ 6Sh)^2}$$

dans lesquelles a*BN, b*BN et L*BN sont les valeurs de a*, b* et L* de la mèche non colorée, a*tém, b*tém et L*tem les valeurs de a*, b* et L* de la mèche colorée avant shampooing, tandis que a*6Sh, b*6Sh et L*6Sh les valeurs de a*, b* et L* de la mèche colorée après 6 shampooings.

Avec la solution $A_1$, la dégradation de la couleur est de 9,19 % après 6 shampooings.
Avec la solution $A_2$, la dégradation de la couleur est de 13,69 % après 6 shampooings.
Avec la solution $A_3$, la dégradation de la couleur est de 19,69 % après 6 shampooings.

Ces résultats montrent que la coloration obtenue avec le procédé conforme à l'invention présente une bonne résistance aux shampooings.

**Exemple 2 :**

1) 0,5 g de la solution $A_1$ est appliqué sur une mèche de cheveux propres et secs. La mèche est ensuite séchée au casque.
2) 0,5 g de la solution C est ensuite appliqué sur la mèche sèche. Sans séchage intermédiaire 0,5 g de la solution B est appliqué.
3) La mèche est ensuite placée 30 minutes à 140 °C.

Le même mode opératoire est mis en oeuvre pour les solutions $A_2$ et $A_3$.
Avec les solutions $A_1$ et $A_2$, les mèches obtenues sont très colorées en rose. Avec la solution $A_3$, la mèche obtenue est très colorée en rouge.

**Exemple 3 :**

1) 0,5 g de la solution $A_1$ est appliqué sur une mèche de cheveux propres et secs. La mèche est ensuite séchée au casque.
2) 0,5 g de la solution B est ensuite appliqué sur la mèche sèche. Sans séchage intermédiaire 0,5 g de la solution C est appliqué.
3) La mèche est ensuite séchée au fer à lisser à 180 °C en restant appuyé 30 secondes sur chaque partie de la mèche.

Le même mode opératoire est mis en oeuvre pour les solutions $A_2$ et $A_3$.
Avec les solutions $A_1$ et $A_2$, les mèches obtenues sont très colorées en rose. Avec la solution $A_3$, la mèche obtenue est très colorée en rouge.

**Exemple 4 :**

1) 0,5 g de la solution $D_1$ est appliqué sur une mèche de cheveux propres et secs. Sans séchage intermédiaire, 0,5 g de la solution C est appliqué.
2) La mèche est ensuite placée 30 minutes à 140 °C.

Le même mode opératoire est mis en oeuvre pour les solutions $D_2$ et $D_3$.
Avec les solutions $D_1$ et $D_2$, les mèches obtenues sont très colorées en rose. Avec la solution $D_3$, la mèche

obtenue est très colorée en rouge.

**Exemple 5 :**

1) 0,5 g de la solution $E_1$ est appliqué sur une mèche de cheveux propres et secs. Sans séchage intermédiaire, 0,5 g de la solution B est appliqué.
2) La mèche est ensuite placée 30 minutes à 140 °C.

Le même mode opératoire est mis en oeuvre pour les solutions $E_2$ et $E_3$.
Avec les solutions $E_1$ et $E_2$, les mèches obtenues sont très colorées en rose. Avec la solution $E_3$, la mèche obtenue est très colorée en rouge.

**Exemple 6 :**

1) 0,5 g de la solution $A_1$ est appliqué sur une mèche de cheveux propres et secs. La mèche est ensuite séchée au casque.
2) 0,5 g de la solution B est ensuite appliqué sur la mèche sèche. Sans séchage intermédiaire, 0,5 g de la solution C est appliqué.
3) La mèche est ensuite placée 2 minutes au casque.
4) 0,3 g d'une solution F fraîchement préparée (moins de 5 minutes) est appliquée sur la mèche. Celle-ci est ensuite séchée au sèche cheveux ou avec un fer à lisser.

Le même mode opératoire est mis en oeuvre pour les solutions $A_2$ et $A_3$.
Avec les solutions $A_1$ et $A_2$, les mèches obtenues sont très colorées en rose. Avec la solution $A_3$, la mèche obtenue est très colorée en rouge.

**Revendications**

**1.** Composition pour la coloration des fibres kératiniques comprenant, dans un milieu cosmétiquement acceptable, au moins un polymère PA portant au moins une fonction chimique A et au moins un polymère PB portant au moins une fonction chimique B, lesdites fonctions chimiques A et B étant aptes à former ensemble des liaisons covalentes, et au moins un pigment.

**2.** Composition selon la revendication 1, dans laquelle les fonctions chimiques A sont choisies parmi les fonctions suivantes :

- époxyde,
- aziridine,
- vinyle et vinyle activée,
- anhydride, chlorure d'acide et esters d'acide carboxylique,
- aldéhydes,
- acétals, hémi-acétals,
- aminals, hémi aminals,
- cétones, alpha-hydroxycétones, alpha-halocétones,
- lactones, thiolactones,
- isocyanate,
- thiocyanate,
- imines,
- imides,
- N-hydroxysuccinimide esters,
- imidates,
- thiosulfate,
- oxazine et oxazoline,

- oxazinium et oxazolinium,
- halogénures d'alkyle en $C_1$ à $C_{30}$ ou d'aryle ou aralkyle en $C_6$ à $C_{30}$ de formule RX avec X = I, Br, CI,
- halogénure de cycle insaturé, carboné ou hétérocycle,
- halogénure de sulfonyle de formule $RSO_2Cl$ ou F, R étant un alkyle en $C_1$ à $C_{30}$.

3. Composition selon la revendication 2, dans laquelle les fonctions chimiques A sont choisies parmi les fonctions anhydride, époxyde, chlorotriazine, aldéhyde, thiosulfate.

4. Composition selon l'une quelconque des revendications précédentes, dans laquelle les fonctions chimiques B sont choisies parmi les fonctions hydroxy, amines primaires et secondaires, les thiols, les acides carboxyliques.

5. Composition selon l'une quelconque des revendications précédentes, dans laquelle le polymère PA porte au moins deux fonctions chimiques A identiques et le polymère PB porte au moins deux fonctions chimiques B identiques.

6. Composition selon l'une quelconque des revendications précédentes, dans laquelle le ou les polymères PA sont choisis parmi les polymères suivants :

- les polymères contenant le motif anhydride,
- les polymères contenant le groupe époxyde et / ou aldéhyde,
- les polysaccharides naturels ou modifiés présentant des fonctions aldéhydes,
- les polysaccharides naturels ou modifiés présentant des fonctions époxy,
- les polymères naturels ou synthétiques à fonctions acide carboxylique.

7. Composition selon l'une quelconque des revendications précédentes, dans laquelle le ou les polymères PB sont choisis parmi les polymères synthétiques ou naturels à fonctions OH ou $NH_2$ ou SH ou COOH.

8. Composition selon la revendication 7, dans laquelle le ou les polymères synthétiques ou naturels à fonctions OH ou $NH_2$ ou SH ou COOH sont choisis parmi les polymères suivants :

- les dendrimères à terminaisons OH, ou $NH_2$ ou SH ou COOH de une ou plusieurs générations,
- les polymères synthétiques à fonction hydroxyle,
- les polyéthylèneimines,
- les polyéthylèneimine thiols, obtenus par réaction de polyéthylèneimines sur la ?-butyrolactone,
- les polyacides aminés présentant des groupes OH, ou $NH_2$ ou SH ou COOH libres,
- les polysaccharides naturels ou modifiés présentant des fonctions OH, ou $NH_2$ ou SH ou COOH.

9. Composition selon l'une quelconque des revendications précédentes, dans laquelle les polymères PA et PB sont choisis parmi les associations suivantes :

- un polymère PB choisi parmi les dendrimères à terminaisons OH, ou $NH_2$ ou SH ou COOH de une ou plusieurs générations en association avec un polymère PA choisi parmi les polymères contenant le motif anhydride ou les polymères contenant le groupe époxyde et / ou aldéhyde,
- un polymère PB choisi parmi les polymères synthétiques à fonction hydroxyle en association avec un polymère PA choisi parmi les polymères contenant le motif anhydride ou les polymères contenant le groupe époxyde,
- un polymère PB choisi parmi les polyéthylèneimine en association avec un polymère PA choisi parmi les polymères contenant le motif anhydride ou les polymères contenant le groupe époxyde et / ou aldéhyde,
- un polymère PB choisi parmi les polyéthylèneimine thiols, obtenus par réaction de polyéthylèneimines sur la ?-butyrolactone, en association avec un polymère PA choisi parmi les polymères contenant le motif anhydride ou les polymères contenant le groupe époxyde et / ou aldéhyde,
- un polymère PB choisi parmi les polyacides aminés présentant des groupes OH, ou $NH_2$ ou SH ou COOH libres en association avec un polymère PA choisi parmi les polymères contenant le motif anhydride ou les polymères contenant le groupe époxyde et / ou aldéhyde,
- un polymère PB choisi parmi les polysaccharides naturels ou modifiés présentant des fonctions OH, ou $NH_2$ ou SH ou COOH en association avec un polymère PA choisi parmi les polymères contenant le motif anhydride ou les polymères contenant le groupe époxyde et / ou aldéhyde,
- un polymère PA choisi parmi les polysaccharides naturels ou modifiés présentant des fonctions aldéhydes en association avec un polymère PB choisi parmi les polymères synthétiques ou naturels à fonctions OH ou $NH_2$ ou SH ou COOH,

- un polymère PA choisi parmi les polysaccharides naturels ou modifiés présentant des fonctions époxy en association avec un polymère PB choisi parmi les polymères synthétiques ou naturels à fonctions OH ou NH$_2$ ou SH ou COOH,
- un polymère PA choisi parmi les polymères naturels ou synthétiques à fonctions acide carboxylique en association avec un polymère PB choisi parmi les polymères synthétiques ou naturels à fonctions OH, NH$_2$ ou SH, en présence d'un carbodiimide, ou d'un acide, d'une base ou d'une enzyme.

10. Composition selon l'une quelconque des revendications précédentes, dans laquelle le ou les polymères PA à fonctions chimiques A sont choisis parmi les polymères suivants :

- Copolymère méthyl-vinyl éther / anhydride maléique,
- PolyMethacrylate de glycidyle,
- Polydiméthylsiloxane de glycidyle,
- Polyamidoamine époxy,
- Epoxy-dextrane,
- Polysaccharides polyaldéhydes obtenus par oxydation de polysaccharides par NaIO$_4$.

11. Composition selon l'une quelconque des revendications précédentes, dans laquelle le ou les polymères PB à fonctions chimiques B sont choisis parmi le polymères suivants :

- Dendrimère PAMAM,
- Dendrimère à fonctions hydroxy,
- PEI (polyéthylène-imine),
- PEI-Thiol,
- Polylysine,
- HP cellulose,
- Amino-dextrane,
- Amino-cellulose,
- PVA (polyvinylacétal),
- Amino PVA,
- Chitosane,
- CM ou HP Dextrane, et
- CM ou HP Chitosane.

12. Composition selon l'une quelconque des revendications précédentes, dans laquelle le ou les pigments sont sous forme de poudre ou de pâte pigmentaire.

13. Composition selon la revendication 12, dans laquelle au moins un pigment est un pigment minéral choisi parmi le dioxyde de titane, traité ou non traité en surface, les oxydes de zirconium ou de cérium, les oxydes de fer ou de chrome, le violet de manganèse, le bleu outremer, l'hydrate de chrome et le bleu ferrique.

14. Composition selon la revendication 12, dans laquelle au moins un pigment est un pigment organique choisi parmi les composés nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.

15. Composition selon la revendication 12, dans laquelle au moins un pigment est un pigment composite composé de particules comportant :

- un noyau inorganique,
- au moins un liant assurant la fixation des pigments organiques sur le noyau, et
- au moins un pigment organique recouvrant au moins partiellement le noyau.

16. Composition selon la revendication 12, dans laquelle au moins un pigment est une laque constituée par un substrat inorganique choisi parmi l'alumine, la silice, le borosilicate de calcium et de sodium ou le borosilicate de calcium et d'aluminium, et l'aluminium sur lequel est adsorbé un colorant.

17. Composition selon la revendication 12, dans laquelle au moins un pigment est un pigment à effets spéciaux choisi parmi les pigments nacrés, les pigments à effets interférentiels non fixés sur un substrat, les pigments fluorescents, les pigments phosphorescents, les pigments photochromiques, les pigments thermochromiques et les quantum dots.

18. Composition selon la revendication 17, dans laquelle le ou les pigments nacrés sont choisis parmi le mica recouvert de titane, ou d'oxychlorure de bismuth, le mica recouvert de titane et d'oxydes de fer, le mica recouvert de titane et de bleu ferrique ou d'oxyde de chrome, le mica recouvert de titane et d'un pigment organique tel que défini à la revendication 14, les pigments nacrés à base d'oxychlorure de bismuth.

19. Composition selon la revendications 17, dans laquelle le ou les pigments à effet interférentiel non fixés sur un substrat sont choisis parmi les cristaux liquides, les paillettes holographiques interférentielles.

20. Composition selon l'une quelconque des revendications précédentes, dans laquelle le ou les pigments sont des pigments colorés.

21. Composition selon l'une quelconque des revendications précédentes, dans laquelle le ou les pigments sont en dispersion.

22. Composition selon l'une quelconque des revendications précédentes, dans laquelle le ou les polymères PA et PB sont chacun présents à des concentrations comprises entre 0,05 et 50 % en poids par rapport au poids total de la composition.

23. Composition selon l'une quelconque des revendications précédentes, dans laquelle le ou les pigments sont chacun présents à des concentrations comprises entre 0,05 et 80 % en poids par rapport au poids total de la composition.

24. Composition selon l'une quelconque des revendications précédentes, comprenant de plus au moins un agent activateur.

25. Composition selon la revendication 24, dans laquelle l'agent activateur est choisi parmi les modificateurs de pH, les co-réactifs et les catalyseurs.

26. Composition selon la revendication 24 ou 25, dans laquelle le ou les agents activateurs sont en dispersion.

27. Composition selon l'une quelconque des revendications 24 à 26, dans laquelle le ou les agents activateurs sont chacun présents à des concentrations comprises entre 0,05 et 30 % en poids par rapport au poids total de la composition.

28. Procédé de coloration des fibres kératiniques, **caractérisé par le fait que** l'on applique sur les fibres kératiniques :

   - au moins un polymère PA et au moins un polymère PB tels que définis à l'une quelconque des revendications 1 à 11 ;
   - et au moins un pigment tel que défini à l'une quelconque des revendication 1 et 12 à 20.

29. Procédé selon la revendication 28, dans lequel le ou les polymères PA, le ou les polymères PB et le ou les pigments sont appliqués sur les fibres kératiniques à partir de plusieurs compositions contenant le ou les polymères PA, le ou les polymères PB et le ou les pigments, seuls ou en mélange, ou à partir d'une seule composition contenant le ou les polymères PA, le ou les polymères PB et le ou les pigments.

30. Procédé selon la revendication 29, dans lequel la composition comprenant le ou les pigments est appliquée avant la ou les compositions comprenant le ou les polymères PA et / ou le ou les polymères PB.

31. Procédé selon l'une quelconque des revendications 28 à 30, dans lequel on augmente la température pour activer la réaction entre le ou les polymères PA et le ou les polymères PB.

32. Procédé selon l'une quelconque des revendications 28 à 30, dans lequel on applique sur les fibres kératiniques au moins un agent activateur tel que défini à la revendication 24 ou 25 pour activer la réaction entre le ou les polymères PA et le ou les polymères PB.

33. Dispositif à plusieurs compartiments, **caractérisé par le fait qu'**il contient au moins deux compositions telles que l'ensemble desdites compositions comprend :

   - au moins un polymère PA et au moins un polymère PB tels que définis à l'une quelconque des revendications 1 à 11 ;
   - et au moins un pigment tel que défini à l'une quelconque des revendication 1 et 12 à 20.

34. Dispositif selon la revendication 33, dans lequel l'ensemble desdites compositions comprend de plus au moins un agent activateur tel que défini à la revendication 24 ou 25.

35. Utilisation pour la coloration des fibres kératiniques d'au moins un polymère PA et d'au moins un polymère PB tels que définis à l'une quelconque des revendications 1 à 11, et d'au moins un pigment tel que défini à l'une quelconque des revendication 1 et 12 à 20.

36. Utilisation selon la revendication 35, dans laquelle la coloration des fibres kératiniques est résistante aux shampooings.

**EP 1 600 149 A1**

<table>
<tr><td colspan="2"><b>Office européen<br>des brevets</b></td><td><b>RAPPORT DE RECHERCHE EUROPEENNE</b></td><td>Numéro de la demande<br>EP 05 29 1149</td></tr>
</table>

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| Y | EP 1 321 126 A (L'OREAL) 25 juin 2003 (2003-06-25) * le document en entier * ----- | 1-36 | A61K7/13 |
| Y | WO 03/020225 A (L'OREAL ET AL.) 13 mars 2003 (2003-03-13) * revendications 1-17 * * page 1, ligne 3 - ligne 5 * * page 2, ligne 17 - page 7, ligne 4 * * page 7, ligne 39 - ligne 43 * * exemples 1,2 * ----- | 1-36 | |

**DOMAINES TECHNIQUES RECHERCHES (Int.Cl.7)**

A61K

Le présent rapport a été établi pour toutes les revendications

| Lieu de la recherche | Date d'achèvement de la recherche | Examinateur |
|---|---|---|
| Berlin | 5 septembre 2005 | Alvarez Alvarez, C |

CATEGORIE DES DOCUMENTS CITES

X : particulièrement pertinent à lui seul
Y : particulièrement pertinent en combinaison avec un
   autre document de la même catégorie
A : arrière-plan technologique
O : divulgation non-écrite
P : document intercalaire

T : théorie ou principe à la base de l'invention
E : document de brevet antérieur, mais publié à la
   date de dépôt ou après cette date
D : cité dans la demande
L : cité pour d'autres raisons
...........................................................................................
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 05 29 1149

La présente annexe indique les membres de la  famille de brevets relatifs aux documents  brevets cités dans le rapport de recherche européenne visé ci-dessus.
Lesdits members sont contenus au fichier informatique de l'Office  européen des brevets à la date du
Les renseignements fournis sont donnés à titre  indicatif et n'engagent pas la responsabilité  de l'Office européen des brevets.

05-09-2005

| Document brevet cité au rapport de recherche | | Date de publication | Membre(s) de la famille de brevet(s) | | Date de publication |
|---|---|---|---|---|---|
| EP 1321126 | A | 25-06-2003 | FR | 2833487 A1 | 20-06-2003 |
| | | | BR | 0207147 A | 30-09-2003 |
| | | | CN | 1426772 A ,C | 02-07-2003 |
| | | | EP | 1321126 A1 | 25-06-2003 |
| | | | JP | 2003192542 A | 09-07-2003 |
| | | | US | 2003143175 A1 | 31-07-2003 |
| WO 03020225 | A | 13-03-2003 | FR | 2829022 A1 | 07-03-2003 |
| | | | EP | 1427382 A1 | 16-06-2004 |
| | | | WO | 03020225 A1 | 13-03-2003 |
| | | | JP | 2005516890 T | 09-06-2005 |
| | | | US | 2003064039 A1 | 03-04-2003 |

EPO FORM P0460

Pour tout renseignement concernant cette annexe :  voir Journal Officiel de l'Office européen des  brevets, No.12/82